(19) Europäisches Patentamt

European Patent Office

Office européen des brevets

(11) **EP 1 325 678 A1**

(12) **EUROPEAN PATENT APPLICATION**
published in accordance with Art. 158(3) EPC

(43) Date of publication:
09.07.2003 Bulletin 2003/28

(51) Int Cl.7: **A01K 67/027**, G01N 33/15, G01N 33/50

(21) Application number: 01974770.8

(22) Date of filing: **10.10.2001**

(86) International application number:
PCT/JP01/08903

(87) International publication number:
WO 02/030185 (18.04.2002 Gazette 2002/16)

(84) Designated Contracting States:
**AT BE CH CY DE DK ES FI FR GB GR IE IT LI LU MC NL PT SE TR**
Designated Extension States:
**AL LT LV MK RO SI**

(30) Priority: **10.10.2000 JP 2000309042**

(71) Applicant: **Japan as represented by President of Niigata University**
**Niigata-shi, Niigata 950-2181 (JP)**

(72) Inventors:
• **NAWA, Hiroyuki**
**Niigata-shi, Niigata 951-8104 (JP)**
• **FUTAMURA, Takashi**
**Niigata-shi, Niigata 950-2002 (JP)**

(74) Representative:
**Wibbelmann, Jobst, Dr., Dipl.-Chem.**
**Wuesthoff & Wuesthoff,**
**Patent- und Rechtsanwälte,**
**Schweigerstrasse 2**
**81541 München (DE)**

(54) **ANIMAL SHOWING SCHIZOPHRENIA-LIKE ABNORMALITY IN COGNITIVE BEHAVIORS AND METHOD OF CONSTRUCTING THE SAME**

(57)   To provide a an animal with schizophrenic sensorimotor and behavioral abnormalities, a specific protein factor inhibiting development of brain function is administrated to a juvenile animal in the stage of its development and an animal exhibiting sensorimotor and behavioral abnormalities is prepared. As the sensorimotor and behavioral abnormalities observed in the animal of the present invention is very similar to schizophrenia, the animal is useful for development of an anti-schizophrenic medicine and a diagnostic agent for schizophrenia.

EP 1 325 678 A1

**Description**

BACKGROUND OF THE INVENTION

1. Field of the invention

This invention relates to schizophrenic model animal with sensorimotor and behavioral abnormalities and a method for preparing the same.

2. Prior art

Schizophrenia is a very serious chronic disease which appears 0.7-1.0% persons of the population. Moreover, in Japan, hundreds of thousand of patients are admitted to a hospital for a long term because of this disease. The main symptom of this disease is accompanied with various psychological disorders, including positive symptoms such as delusion, hallucination and auditory hallucination, in addition to negative symptoms such as withdrawal from society and depression. At present, the cause of occurrence of this disease and the biological pathology of this disease are not elucidated.

Schizophrenia occurs from adolescence to manhood with characteristic symptoms in perception, cerebration, emotion and behavior. In many cases, this disease progresses chronically and the patients suffer from various difficulties for adaptation to society. With regard to schizophrenia, there are classifications of positive symptoms (hallucination, delusion, diminished cerebration, tension, curious behavior, etc.) and negative symptoms (flattening in motion, decrease in will and social withdrawal, etc.). Socially, because of the specific pathology of this disease, establishment of a consistent and comprehensive treatment system against this disease, such as detection of occurrence, treatment and social reversion at early stage, and prevention of recurrence, has been desired.

As an effective therapeutic medicine to improve the positive symptom of schizophrenia, only antagonists to neurotransmitters, such as dopamine antagonist or serotonin antagonist are known. In many cases, long period of administration of such medicament is indispensable. In concrete, the examples of medicaments conventionally prescribed to patients are phenothiazine derivatives, thioxanthene derivatives, butyrophenone and benzamide derivatives.

In addition to investigation on the mechanism of action of these medicaments, it is known that stimulant drugs such as amphetamine induce positive phenomenons of schizophrenia in human beings. Therefore, the "dopamine hypothesis" that the functional abnormality of dopamine is involved in occurrence of schizophrenia has been presented. Moreover, from these facts, an animal, to which amphetamine has been administrated chronically, can be adopted as a model animal of schizophrenia. Similarly, a medicament that induces hallucination in human beings can be administrated to an animal to prepare a model animal for schizophrenia. An animal, to which phencyclidine administrated, is included in one of such example. Here, phencyclidine is an inhibitor of glutamine receptor and it has been recognized that glutamine receptor is involved in physiological function of brain, such as memory and study.

As to most of the conventional schizophrenic model animal mentioned above, they often exhibit transient brain function disorder depending on the administration of the medicaments. However, they can not reproduce chronic pathology of schizophrenia observed in human beings. Moreover, though the dopamine antagonists can improve the positive symptom, only few therapeutic medicaments are effective for treatment of negative symptoms. It is considered that such situation is due to lack of a suitable schizophrenic animal model.

Until now, various hypothesis have been proposed on the mechanism on the onset of schizophrenia. One of such is the brain development disorder hypothesis (Reference 1) presented by Winberger et al. However, as to the biological factor that causes abnormality in development of cerebral nerve system and the mechanism involved in the occurrence of such abnormality, elucidation has not been performed yet. The present invention firstly provides a model animal that scientifically embodies this hypothesis.

SUMMARY OF THE INVENTION

Therefore, the object of the present invention is to provide a model animal with persistent sensorimotor and behavioral abnormalities extremely similar to schizophrenia, to be utilized in investigation of the mechanism of schizophrenia and in development of a therapeutic medicine.

To solve the above-mentioned problems, the present inventors noticed on the above-mentioned brain development disorder hypothesis. That is, the present inventors prepared a model animal with persistent sensorimotor and behavioral abnormalities extremely similar to schizophrenia, by administering specific protein factors which inhibit development of brain function or by expressing the genes encoding the protein factors.

This invention is explained in detail in the following description, however, the preferred embodiments and the examples are not to be considered to limit the range of this invention.

BRIEF DESCRIPTION OF THE DRAWINGS

Fig. 1 is a graph showing prepulse inhibition measured on the control group and on the EGF group.

Fig. 2 is a graph showing alteration of pre-pulse inhibition in accordance to postnatal day.

Fig. 3 is a graph showing the effect of clozapine injection on prepulse inhibition, measured on control group and EGF-treated group.

Fig. 4 is a graph showing the effect of clozapin injection and haloperidol injection on prepulse inhibition and startle response, measured on control group and EGF-treated group.

Fig. 5 is graph showing the abnormality in motor activity by administration of EGF, measured on the rats of postnatal day 52 and postnatal day 24.

Fig. 6 is a graph showing the abnormality in motor activity by administration of EGF and improvement of the abnormality by clozapin and haloperidol, measured on the rats of postnatal day 52 and postnatal day 24.

Fig. 7 is a graph showing the abnormality in social interaction by administration of EGF, as the sniffing behavior as an index.

Fig. 8 is a graph showing the effect of EGF administration on learning ability of rats by the measurement of active avoidance response.

DESCRIPTION OF THE PREFERRED EMBODIMENTS

The present invention relates to a schizophrenic model animal with sensorimotor and behavioral abnormalities by administration of specific protein factors which inhibit development of brain function to a juvenile animal in the stage of its growth, and a method to prepare the animal. The animal of the present invention can be prepared not only by administration of the above-mentioned protein factor but also by expression of the genes encoding the protein factors. Epidermal growth factor (EGF), tumor growth factor alpha (TGF-alpha), heparin-binding epidermal growth factor and amphiregulin are preferred as the protein factors to be adopted. Actual examples of the sensorimotor and behavioral abnormalities caused by epidermal growth factor (EGF) are shown in the following examples. Three protein factors of tumor growth factor alpha (TGF-alpha), heparin-binding epidermal growth factor and amphiregulin have similar steric structure in common. Moreover, it has been known that all of them bind to the receptor molecule of epidermal growth factor, thus exhibit similar biological activities (Literature 5). The detailed mechanism of the above-mentioned protein factors to cause inhibition of growth of brain function has not yet been elucidated. Meanwhile, these protein factors have been known to activate a series of STAT (signal transducers and activators of transcription) pathway in the signal transduction system of the cell. Therefore, it is assumed that the STAT pathway is involved in the sensorimotor and behavioral abnormalities of the model animal according to the present invention. The protein factors to be used in the present invention are not limited only to the above-mentioned protein factors, and other protein factors having some neurochemically equivalent function can be also adopted for the purpose of this invention.

These protein factors needs to be administrated or expressed at the developing stage of these animals and the stage should be an embryo or a fetus in pregnancy or a stage as early as possible after birth. In the present specification, the phrase of "a juvenile animal in the stage of its development" means an animal wherein the nerve system of the animal is in the stage development thus has not yet been completed. In the juvenile animal with its development of the neuron not completed, the protein factors exhibit significant effect to inhibit brain function. For example, in the case of murine, the development of brain is substantially completed at postnatal day 20-30, in respect of the weight of the brain. Therefore, the murine prior to this term is designated by the term. In the present invention, administration or expression of the protein factor needs to be completed prior to the term.

Moreover, as the species of the animals to be used as the model animal of the present invention, the method of the present invention can be applied to any of mammals except for human being. As the preferred animal species, there may be mentioned monkey, chimpanzee, dog, cat, rabbit, guinea pig, rat and mouse. Particularly preferred animal species are rat, mouse, chimpanzee and monkey, since many data have been accumulated so far.

As a means to administrate or express these protein factors, two kinds methods can be mentioned. One is the direct injection of these protein factors, by peritoneal injection or subcutaneous injection of the animal. As a protein factor is easily decomposed in a digestive tract, such compound is not suited for oral administration. The dose of the protein factor alters depending on the kind of the protein factor, the kind of the animal or the route of administration. However, in general, the administrated dose per day is in the range of 0.01 mg/kg to 100 mg/kg, preferably 0.1 mg/kg to 10 mg/kg. In the case of administration as a protein factor, the protein factor can be metabolized and decomposed in a body. Then the concentration in the body is rapidly decreased, thus it should be injected plural times for several days. As to origin of these protein factors, those obtained by massive production in bacteria according to the method of gene recombination or those purified from animal cells can be used.

Another method utilizes excessive expression of the protein factor, using genes encoding these protein factors. With regard to epidermal growth factor (EGF), tumor growth factor alpha (TGF-alpha), heparin-binding epidermal growth factor and amphiregulin, genes encoding these factors have already been known, thus excessive expression of the protein factor can be achieved, using various methods conventionally used in this art. That is, the gene encoding the above-mentioned protein factor can be injected to the targeted portion of the animal, for example, cerebral ventricles, whereby excessive expression of the protein is forced. Then model animal according to the present invention with sensorimotor and behavioral abnormalities can be prepared. To achieve local expression of these genes, the method using recombinant virus vector, the calcium phosphate co-precipitation wherein DNA is introduced into cells with the fine crystals of calcium phosphate, the electroporation method wherein fine pores are opened on the cell membrane by applying transient high voltage, the DEAE dextran method, the lipofection method wherein DNA is sealed in the artificial lipid membrane to fuse with a cell membrane and the like, can be used. Moreover, the particle gun method, wherein DNA bound to gold particles is shot into the tissue with a specific gun, has been known. In these methods, the gene is introduced into a cell. As a result, the effects achieved by the methods are equal to those obtained by administration of the protein factor.

Moreover, the model animal of the present invention can be prepared by operating an embryonic germ or a fertilized egg of an animal, whereby genetic trait of the animal can be transformed and the above-mentioned protein factor can be expressed excessively. As a means conventionally used to attain such purpose, the microinjection method and the embryonic stem cell (ES cell) method can be mentioned. In the microinjection method, DNA is directly injected into the fertilized egg using a fine glass tube under microscopic observation. In the ES cell method, the objective DNA is introduced into the ES cell and then the ES cell is returned to an embryonic germ. According to these methods, once the transgenic animal is prepared, the line can be maintained and the model animal for schizophrenia can be supplied.

The model animal according to the present invention is particularly useful as a tool for development of a therapeutic medicine or a diagnostic agent of psychosis showing schizophrenic pathology. Moreover, the model animal is also useful as a tool for elucidation of the mechanism of psychosis showing pathological feature of schizophrenia or similar to schizophrenia. As already mentioned above, development of a therapeutic medicine for schizophrenia is not sufficient at present. In particular, a medicine having significant effect for improvement of the negative symptom has not been developed yet, because of lack of a good model animal. Thus, the model animal of the present invention will play a remarkable role to solve such a problem. Moreover, the model animal of the present invention prepared based on the nerve development disorder hypothesis would be extremely useful for the purpose to elucidate the biochemical mechanism of schizophrenia. In the present specification, a method to use the animal prepared according to the present method as a schizophrenic model animal with sensorimotor and behavioral abnormalities means a method to use the animal as a tool to develop a therapeutic medicine or diagnostic agent of a psychosis showing pathological feature of schizophrenia or that similar to schizophrenia, or as a tool to elucidate biochemical mechanism of schizophrenia. However, the range of this invention is not limited to them, and other possible usage realized by using the animal with sensorimotor and behavioral abnormalities according to this invention should be also included within the scope of this invention.

The model animal of schizophrenia prepared by the method of this invention exhibits persistent sensorimotor and behavioral abnormalities like those observed in schizophrenic patients, after nerve development is completed in the animal. It seems to be consistent to the phenomenon that onset of schizophrenia frequently occurs at or after adolescence. In the followings, the methods utilized to evaluate the sensorimotor and behavioral abnormalities of the model animal according to the present invention is described. Incidentally, in the present specification, "persistent sensorimotor and behavioral abnormalities" means that the abnormalities are not transient and persist long term at least ten days.

As the method used to evaluate the sensorimotor and behavioral abnormalities, behavioristic measurements such as prepulse inhibition of startle response, latent inhibition, social interaction, motor activity of the animal, can be mentioned. The prepulse inhibition of startle response is a test to evaluate the ability of perception - motor activity, using the startle response as an index and the startle response can be evaluated in human being and animal in common. In this test, evaluation of attention deficit and abnormality in information processing in brain can be achieved scientifically and objectively. These are considered to be the main factors involved in schizophrenic pathology. When a weak acoustic stimulant (prepulse), which does not cause a startle response, is given to an animal for 30-150 msec prior to subjecting to a large sound of 120db, the startle response caused by the large sound decreases. This test is carried out by measurement of such reaction. Such decrease caused by the prepulse is called "prepulse inhibition". It has been known that significant decrease in the prepulse inhibition is observed in schizophrenic patients and in schizophrenic model animals (Literature 2).

In latent inhibition, inhibition of learning caused by "practice", which was performed prior to the learning test, is evaluated. The principle of the test is similar to that of prepulse inhibition described above (Reference 3). For example, in a Pavlov type conditioning study, if the test animal is previously accustomed with the bell stimulus (conditioning stimulus : CS) without feeding (unconditioned stimulus : US), the learning of "bell (CS) means feeding (US)" is inhibited.

In general, in a schizophrenic patient or its model animal, the ability to pay overall attention and to learn by "practice" are decreased. Therefore, learning inhibition due to previous presentation of CS and latent inhibition hardly occurs in the schizophrenic patients or in its model animals (Reference 3).

Social interaction test is a test that makes an index of the schizophrenic patient's trait that they dislike to make contact with other individuals and keeps away from society, can be made to an index. In general, the sniffling behavior of the animals, usually observed when an animal meets to an unknown individual, is observed. In general, in schizophrenic patients and in schizophrenic model animals, decrease of this behavior has been reported.

(Example)

1. Persistent abnormality of prepulse inhibition by administration of epidermal growth factor

SD rats (Nippon SLC) from postnatal day 2 were used as the test animals. Human recombinant epidermal growth factor (EGF; Higeta Shoyu) and cytochrome-C (Sigma) were dissolved in physiological saline at 70 µL/mL. From postnatal day 2, 25 µL of the solution per 1g of the body weights of rats (each 1.75 mg/kg) was subcutaneously administered to the rat at the nape of the neck every other day. This operation was repeated five times until postnatal day 10. From postnatal day 21, the startle response and the prepulse inhibition were measured in a startled chamber (San diego Instruments). That is, acoustic stimulus was used as the sensory stimulation to induce startle response. As a prepulse, acoustic stimuli at the intensity 5 to 15 db above the environmental noise (background noise) was given. After 100 milliseconds, a pulse stimuli at the intensity of 120 db was given. The extent of the response obtained under combination of prepulse and 120 db was divided by that obtained under 120 db alone (%) to obtain response ratio. The response ratio was subtracted from 100 and it was designated prepulse inhibition (PPI). The calculation formula used to calculate the prepulse inhibition (PPI) is shown below.

$$PPI(\%) = \frac{(\text{Extent of response at 120 db alone}) - (\text{Extent of response with combination of pre-pulse})}{\text{Extent of response at 120 db alone}} \times 100$$

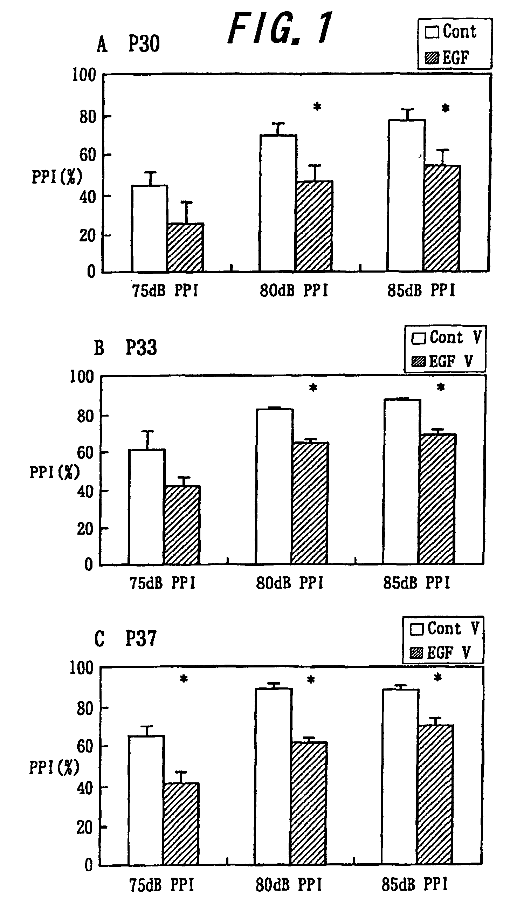

At postnatal day 30 (P30), postnatal day 33 (P33), postnatal day 37 (P37), the prepulse inhibitions (PPI) were measured (Fig. 1). In Fig. 1, Fig. 1A shows the result of prepulse inhibition at postnatal days 30 (P30), Fig. 1B shows the result of prepulse inhibition at postnatal day 33 (P33), and Fig. 1C shows the result of prepulse inhibition at postnatal day 37 (P37), respectively. In P30, the EGF administrated group (EGF V) showed significant decrease (* $p<0.05$) in the prepulse inhibition, as compared with the cytochrome-C administered group (Cont V) at the prepulse intensities of 80 db and 85 db. In P33, the EGF administrated group (EGF V) also showed significant decrease (* $p<0.05$) in the prepulse inhibition at the prepulse intensities of 80 db and 85 db. In P37, the EGF administrated group (EGF V) showed significant decrease (* $p<0.05$) in prepulse inhibition at all of the prepulse intensities of 75 db, 80 db and 85 db.

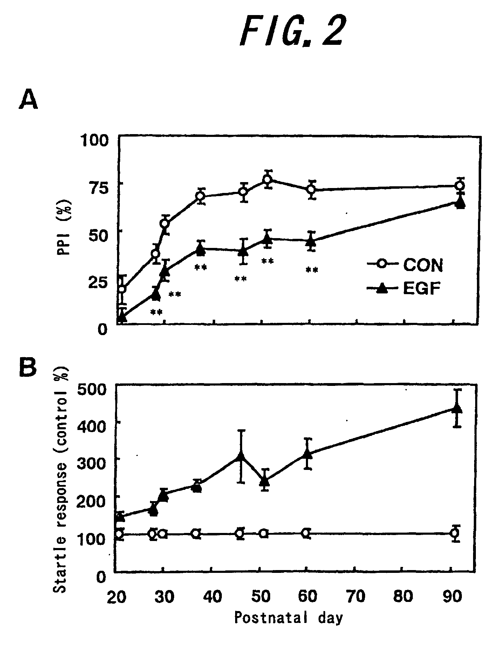

The results of the prepulse inhibition (Fig. 2A) and the startle response (Fig. 2B) analyzed in accordance to postnatal days are shown in Fig. 2. In Fig. 2, the open circle indicate the results cytochrome-C administered control group (n=10) and the closed triangle indicate the epidermal growth factor administrated group (n=10), respectively. In Fig. 2A, the measurements were performed under the prepulse intensity of 75db at the postnatal days 21, 28, 30, 37, 46, 51, 60 and 90. The results were indicated by mean ± SEM and the asterisk marks indicate significant difference (* $p<0.05$, **$<0.01$) between the two groups. The startle response shown in Fig. 2B indicates difference between the control rats and EGF rats, in response to the acoustic stimuli of 120db (control %). In the results of Fig. 2, the significant difference was recognized after postnatal day 28.

2. Improved abnormality in prepulse inhibition by clozapine administration

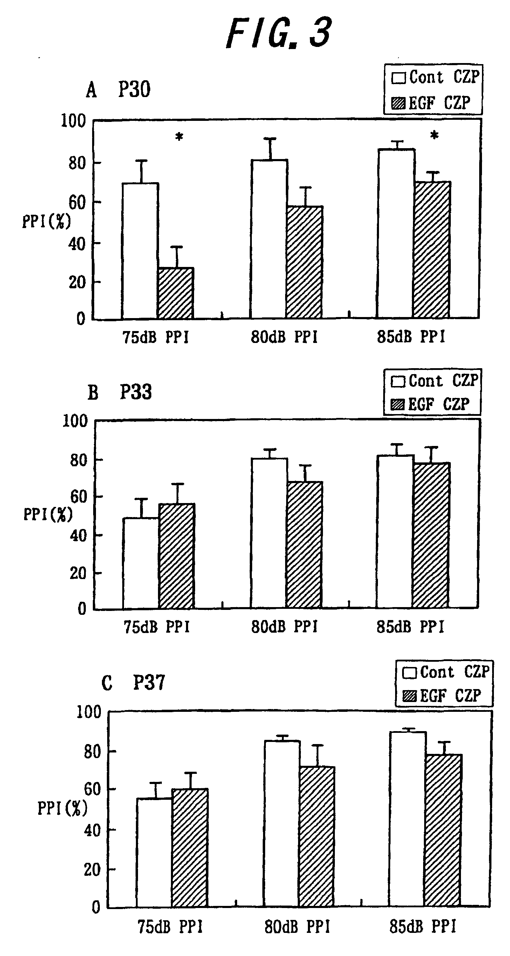

The effect of 8-Chloro-11-(4-methyl-1-piperazinyl)-5H-dibenzo[b,e][1,4]-diazepine (clozapine : Sigma-Aldrich Fine Chemical Co.Ltd.), recognized to be a therapeutic agent for schizophrenia of human beings, was evaluated on the present model animal to evaluate the adequacy of this model. To the human recombinant EGF administered group and the control group (cytochrome-C administered), clozapine was peritoneally administrated everyday. This administration was performed from postnatal day 21 and chronic abnormality of prepulse inhibition firstly occurs around this period. The prepulse inhibition (PPI) was measured at the postnatal days 30 (P30), 33 (P33) and 37 (P37) (Fig. 3). The result at postnatal day 30 (P30) is shown in Fig. 3A, the result at postnatal day 33 (P33) is shown in Fig. 3B, and the result at postnatal day 37 (P37) is shown in Fig. 3C, respectively. At postnatal day 30 (P30), which is the 9th day of clozapine treatment; significant abnormality in the prepulse inhibition remained in the EGF administered group (EGF CZP) as compared with the cytochrome-C administered group (Cont CZP) (Fig. 3A). However, at the period of postnatal days 33 (P33) and 37 (P37), significant difference in prepulse inhibition of the EGF administered group (EGF CZP) disappeared as compared with the cytochrome-C administered group (Cont CZP) (Fig. 3B, Fig. 3C).

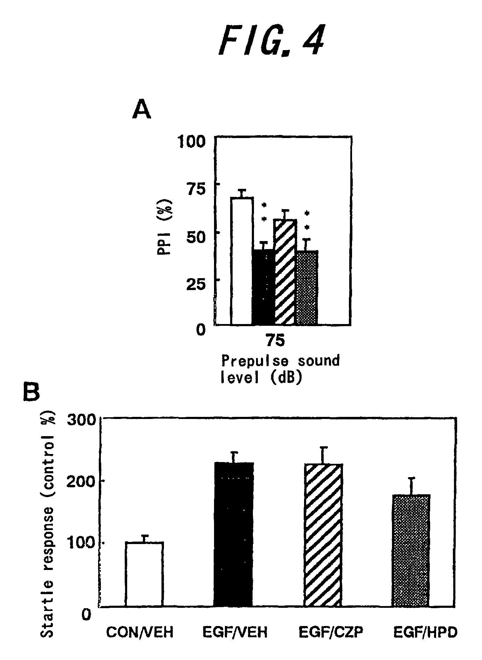

Moreover, the effect of 4-(4-[p-Chlorophenyl]-4-hydroxypiperidino)-4'-fluorobutyrophenone (Haloperidol : Sigma-Aldrich Fine Chemical Co.Ltd.) was also evaluated. Haloperidol was peritoneally administered at a dose of 0.3 mg/kg. In Fig. 4, investigation was performed on the four groups of: (1) the control rats group (CON/VEH) with saline administration (as a vehicle), (2) the EGF administered / saline injected group (EGF/VEH, n=10), (3) the EGF administered / clozapin injected group (EGF/CLP, n=10) and (4) the EGF administered / haloperidol injected group (EGF/ HPD, n=6). The administration of saline, clozapine and haloperidol continued for one week. Twenty-three hours after the final administration, the test was performed on the rats of the respective group. Fig. 4A shows the data of the prepulse inhibition at the prepulse intensity of 75db, and the asterisk marks indicate significant difference as compared with the CON/VEH group (* p<0.05, ** p<0.01). In Fig. 4A, clozapine administration showed significant improvement in prepulse inhibition. However, such effect was not observed on haloperidol. Fig. 4B shows the extent of startle response (% in comparison with CON/VEH group) of each group against the acoustic stimuli of 120db. The EGF administered groups showed startle response significant stronger than the CON/VEH group, however, the anti-psychopathic medicines described above were not effective for the startle response at 120db.

3. Spontaneous abnormality in motor activity due to administration of EGF

The locomotor activities of the animals were measured at postnatal days 24 and 52. At postnatal day 24, it was measured using automated Activity Monitor (Neuro Science). At postnatal day 53, using a box having the size of 51 x 51 x 80 cm with lines of every 17 cms, the number of line crossing (horizonal activity) and the number of learing counts (vertical activity) within 10 minutes were measured.

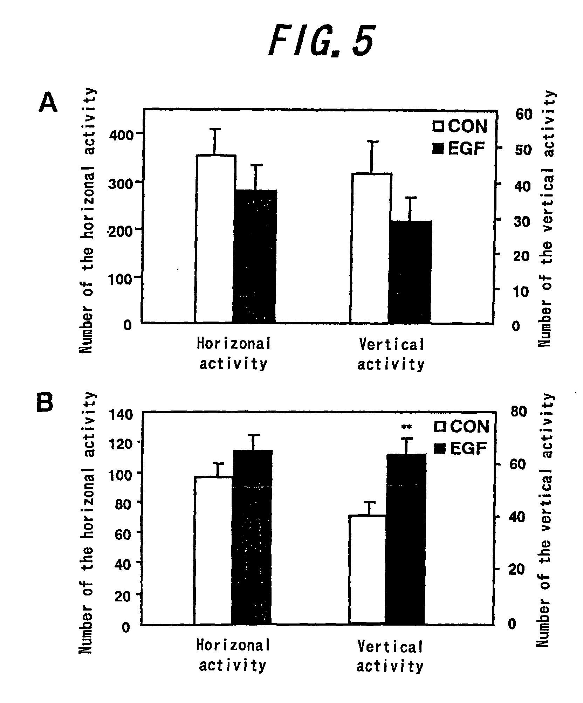

The mean numbers (±SEM) of horizonal activity (Line Cross) and vertical activity (Rearing) at postnatal days 24 (P24) and 52 (P52) are shown in Fig. 5. Fig. 5A is the result obtained at postnatal day 24 (n=15, 8 males and 7 females) and Fig. 5B is the result obtained at postnatal day 52 (n=10, 5 males and 5 females). In Fig. 5, the left vertical axis indicates the number of the horizonal activity, while the right vertical axis indicates the number of the vertical activity. The left columns indicate the number of line crossing and the right columns indicate the number of learing counts, respectively. Moreover, the white columns indicate the control group (CON) and the black columns indicate the epidermal EGF administered group, respectively.

At postnatal day 24, the EGF administered group showed no significant difference as compared with the cytochrome-C administered group (CON), both in the number of the horizonal activity and in the number of the vertical activity (Fig. 5A). After further growth, at postnatal day 52 of fecundity acquisition, significant difference was observed on the number of vertical activity of the EGF group, as compared with the cytochrome-C administered group (Fig. 5). This data corresponds to the knowledge that schizophrenia frequent occurs after adolescence.

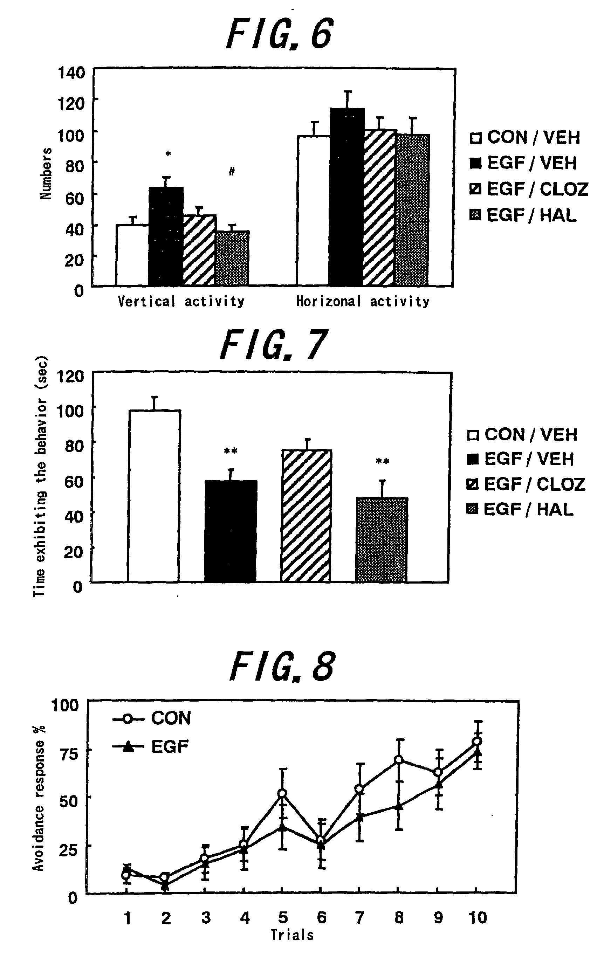

The effect of anti-psychotic medicine on motor activity was also investigated using rats of postnatal day 52. The results investigated on four groups are shown in Fig. 6, namely 1) the control rats group (CON/VEH) with saline administration as a vehicle, (2) the EGF administrated / saline injected group (EGF/VEH, n=10), (3) the EGF administered / clozapin injected group (EGF/CLP, n=10) and (4) the EGF administered / haloperidol injected group (EGF/ HAL, n=6). The same numbers of male and female are used in each of the groups. Saline, haloperidol and clozapine were administered for three weeks, and each group was tested 48 hours after the final administration. Moreover, the asterisk mark (*) and the sharp mark (#) indicate significant difference as compared with the CON/VEH group and EGF/VEH group, respectively (* p<0.05, # p<0.05). As shown in Fig. 6, the vertical activity of the EGF rat groups decreased significantly by chronic administration of haloperidol or clozapine. Meanwhile, administration of these medicines was not effective on the horizonal activity.

4. Social interaction abnormality by administration of epidermal growth factor

Moreover, the abnormality in social interaction of the EGF administered rats was investigated according to the method of File et al. Using two rats of postnatal day 52, sniffling behavior between two rats was observed and the time of sniffling behavior was used as an index of anxiety. Under the condition employed in experiments to evaluate the social interaction, both of the control rat group and the EGF administered rat group exhibited the avoidance behavior. In concrete, the sniffling behavior was observed under high light level and unfamiliar condition in the opened space. The both groups of animals are videotaped and sniffing behaviors of the animals against those of the partner was recorded (Fig. 7).

In Fig. 7, the mean times (±SEM) of the sniffing behavior were shown. The investigation was performed on the four groups of (1) the control rats group (CON/VEH) with saline administration as a vehicle, (2) the EGF administrated / saline injected group (EGF/VEH, n=10), (3) the EGF administered / clozapin injected group (EGF/CLOZ, n=10) and (4) the EGF administered / haloperidol injected group (EGF/HAL, n=6). The EGF administrated rats were pretreated by clozapine or haloperidol for three weeks and the test was performed after 48 hours of the final injection on each group. The asterisk marks indicate significant difference as compared with the CON/VEH group (** $p<0.01$). In comparison with the control rats, the sniffing behavior against the unfamiliar partner reduced significantly (EGF/VEH). Moreover, the reduced sniffing behavior in the EGF adinistrated group recovered by injection of clozapine (EGF/CLOZ). Meanwhile, haloperidol was not effective to recover the sniffing behavior (EGF/HAL).

5. Investigation on the learning ability by active avoidance response

Furthermore, the learning ability was investigated by active avoidance response. The active avoidance test was performed in a shuttle box, using rats of postnatal day 51-60 (10 trials / day). The conditioning stimulus (CS) adopted here was a stimuli by noise of 80dB tone and room light for Ss. When the CS was on, the test animals had to cross the other side of the shuttle box apparatus (avoidance response) in order to turn it off and avoid the appearance of the unconditioned stimulus (US) described below. As the US, an electric shock by conducting 0.6-mA intensity of constant current for 10s was utilized. Moreover, the test animals were investigated whether they are capable of avoiding the electrical shock by learning.

In Fig. 8, the percentages (±SEM) of the avoidance response are shown. In Fig. 8, the open circle symbols indicate cytochrome-c treated control group (CON : n=10), and the closed triangle symbols indicate EGF-treated group (EGF : n=10), respectively. The ability of both groups to avoid the electric shock was significantly improved during training and there was no difference between the both groups in ability to escape from the electric shock. There was also no difference between these two groups in the latency of response or the number of crossings between the two sides of the shuttle box. Thus, the learning ability of the EGF-treated rats appeared to be normal in the adult phase, ruling out possibility that all of their brain function were impaired.

According to the present invention, a schizophrenic model animal with sensorimotor and behavioral abnormalities was prepared, by administration of a specific protein factor inhibiting development of brain function. The animal of the present invention will enable development and evaluation of anti-schizophrenic medicines and diagnostic agents for schizophrenia.

References

1. Weinberger DR. Arch. Gen Psychiatry 44: 660-669 (1987)
2. Braff DL, Geyer MA. Arch Gen Psychiatry 47: 181-188 (1990)
3. Brauch I, Hemsley DR, Gray JA, J. Nerv. Ment. Dis. 176: 598-606 (1991)
4. Sams-Dodd F.Rev Neurosci. 10(1):59-90. (1999).
5. Morrison R; "Neurotrophic Factors" Loughlin SE and Fallon JH eds. Academic Press, Chapter 11; 339-357 (1993)

**Claims**

1. A method for preparation of an animal with persistent sensorimotor and behavioral abnormalities, by plural or continuous administration of a protein factor selected from the group consisting of Epidermal Growth factor, Tumor Growth factor alpha, Heparin-binding Epidermal Growth factor and Amphiregulin to a juvenile animal in the stage of its development.

2. An animal with persistent sensorimotor and behavioral abnormalities prepared by the method according to Claim 1.

3. A method to use the animal prepared by the method according to Claim 1, as a model animal with persistent sensorimotor and behavioral abnormalities.

4. A method for preparation of an animal with persistent sensorimotor and behavioral abnormalities, the method comprising the steps of:

   (1) local incorporation of a gene encoding a protein factor selected from the group consisting of Epidermal Growth factor, Tumor Growth factor alpha, Heparin-binding Epidermal Growth factor and Amphiregulin to a juvenile animal in the stage of its development; and
   (2) expression of said protein factor in the body of said animal.

**5.** An animal with persistent sensorimotor and behavioral abnormalities prepared by the method according to Claim 4.

**6.** A method to use the animal prepared according to the method according to Claim 4, as a model animal with persistent sensorimotor and behavioral abnormalities.

**7.** A method for preparation of an animal with persistent sensorimotor and behavioral abnormalities, the method comprising the steps of:

(1) incorporation of a gene encoding a protein factor selected from the group consisting of Epidermal Growth factor, Tumor Growth factor alpha, Heparin-binding Epidermal Growth factor and Amphiregulin to an early embryonic germ or a fertilized egg of an animal to transform said embryonic germ or said fertilized egg of said animal; and
(2) development of an animal individual from said embryonic germ or said fertilized egg to achieve expression of said protein factor in said animal individual.

**8.** An animal with persistent sensorimotor and behavioral abnormalities prepared by the method according to Claim 7.

**9.** A method to use the animal prepared according to the method according to Claim 7, as a model animal with persistent sensorimotor and behavioral abnormalities.

FIG. 1

# FIG. 2

# FIG. 3

# FIG. 4

# FIG. 5

## FIG. 6

## FIG. 7

## FIG. 8

# EP 1 325 678 A1

<table>
<tr><td colspan="2"><b>INTERNATIONAL SEARCH REPORT</b></td><td>International application No.<br><br>PCT/JP01/08903</td></tr>
</table>

| A. CLASSIFICATION OF SUBJECT MATTER |
|---|
| Int.Cl$^7$  A01K67/027, G01N33/15, G01N33/50 |

According to International Patent Classification (IPC) or to both national classification and IPC

| B. FIELDS SEARCHED |
|---|

Minimum documentation searched (classification system followed by classification symbols)
   Int.Cl$^7$   A01K67/027, G01N33/15, G01N33/50

Documentation searched other than minimum documentation to the extent that such documents are included in the fields searched
   Jitsuyo Shinan Koho          1922-1996    Toroku Jitsuyo Shinan Koho  1994-2001
   Kokai Jitsuyo Shinan Koho    1971-2001    Jitsuyo Shinan Toroku Koho  1996-2001

Electronic data base consulted during the international search (name of data base and, where practicable, search terms used)
   DIALOG (BIOSIS)
   JOIS(JICST FILE)

| C. DOCUMENTS CONSIDERED TO BE RELEVANT |
|---|

| Category* | Citation of document, with indication, where appropriate, of the relevant passages | Relevant to claim No. |
|---|---|---|
| A | ERWIN et al.,<br>Intestinal overexpression of EGF in transgenic<br>mice enhances adaptation after small bowel resection.<br>American Journal of Physiology(Gastrointestinal and<br>Liver Physiology) 1999, vol.277, Issue 3, pp.G533-G540 | 1-9 |
| X | WONG et al.,<br>Overexpression of Epidermal Growth Factor<br>Induced Hypospermatogenesis in Transgenic Mice.<br>THE JOURNAL OF BIOLOGICAL CHEMISTRY.  16 June, 2000,<br>Vol.275, No.24, pages 18297-18301 | 2,5,7,8 |
| A | TROPEPE et al.,<br>Distinct Neural Stem Cells Proliferate<br>in Response to EGF and FGF in the Developing Mouse<br>Telencephalon. Developmental Biology, 1999, Vol.208<br>pages 166-188. | 1-9 |
| P,X | CHAN et al.,<br>Expression of Epidermal Growth Factor in<br>Transgenic Mice Causes Growth Retardation.THE JOURNAL<br>OF BIOLOGICAL CHEMISTRY. 08 December, 2000, Vol.275, | 2,5,7,8 |

| ☒ Further documents are listed in the continuation of Box C. | ☐ See patent family annex. |
|---|---|

| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search<br>   08 January, 2002 (08.01.02) | Date of mailing of the international search report<br>   22 January, 2001 (22.01.02) |
|---|---|
| Name and mailing address of the ISA/<br>   Japanese Patent Office | Authorized officer |
| Facsimile No. | Telephone No. |

Form PCT/ISA/210 (second sheet) (July 1992)

15

## INTERNATIONAL SEARCH REPORT

| | International application No. |
| --- | --- |
| | PCT/JP01/08903 |

C (Continuation).    DOCUMENTS CONSIDERED TO BE RELEVANT

| Category* | Citation of document, with indication, where appropriate, of the relevant passages | Relevant to claim No. |
| --- | --- | --- |
| | No.49, pages 38693-38698 | |
| A | NAKAGAWA et al., Neuronal and glial expression of heparin-binding EGF-like growth factor in central nervous system of prenatal and early postnatal rat. DEVELOPMENTAL BRAIN RESEARCH, 1998, Vol.108, pages 263-272 | 1-9 |
| A | TRAVERSE et al., EGF triggers neuronal differentiation of PC12 cells that overexpress the EGF receptor. Current Biology, 1994, Vol.4, No.8, pages 694-701 | 1-9 |
| A | FALLON et al., Epidermal Growth Factor Immunoreactive Material in the Central Nervous System:Location and Development. Science, 1984, Vol.224, No.4653, pages 1107-1109 | 1-9 |
| A | HORIGUCHI et al., Shinkei-kan Saibou Shinkei Saisei ni okeru Cytokine no Yakuwari, Jikken Igaku, 20 September, 2000, Vol.18, No.15, pages 2135-2141 | 1-9 |

Form PCT/ISA/210 (continuation of second sheet) (July 1992)